# EUROPEAN PATENT SPECIFICATION

(11) **EP 0 421 541 B1**
(45) Date of publication and mention of the grant of the patent: **24.05.1995**
(21) Application number: 90202610.3
(22) Date of filing: 02.10.1990
(51) Int. Cl.: A61K 9/44, A61K 9/20, A61J 3/10

(54) **Multi-fractionable tablet structure**
Mehrfach teilbare Tablettenstruktur
Structure de comprimé pulvérisable

(30) Priority: 04.10.1989 US 416902
(43) Date of publication of application: 10.04.1991
(73) Proprietor: Akzo Nobel N.V., 6824 BM Arnhem (NL)
(72) Inventor: Becker, John W., Littleton, CO 80122 (US)
(74) Representative: Hermans, Franciscus G.M.

(56) References cited:
- GB-A- 2 047 095

## Description

The present invention relates to a tablet structure comprising two planar surfaces constituting a unitary dose, which structure is readily dividable into two or three equal sub-dosage units through scoremarkings.

It is well known in the art that tablets may comprise grooves or scoremarkings to facilitate breakage. Circular tablets easily severable into halves are described in U.S. patent no. 3.883.647. A circular tablet said to be dividable into quarters is disclosed in U.S. patent no. 3.723.614 which tablet comprises scoremarkings resembling a maltese cross.

In the U.S. patent no 4.215.104 an approximately rectangular tablet is disclosed, which comprises one or two transverse scoremarkings on each opposite side and two, respectively one transverse scoremarking on the two sides connecting the opposite sides. In one embodiment the rectangular tablet comprises one longitudinal scoremarking on one side and two transversely positioned scoremarkings on the opposite side.

These diverse attempts to improve the convenience and accuracy of breaking a grooved tablet have achieved limited success only.

Circular tablets are frequently difficult to grasp for purposes of breaking.

This problem can be overcome partially but not completely with the rectangular tablets of US patent no. 4.215.104.

If doses equivalent to one third of the tablet are to be administered, some difficulties arise. After breaking off the first third, the length of the tablet is reduced to two thirds of the original length. This makes the tablet more difficult to handle for lack of grip. A typical attempt to sever the last two thirds of the tablet is by means of a sharp knife, which on the one hand often results in fracture of the tablet into undesirable small pieces and which on the other hand often results in both sections being propelled away from the initial location because of the pressure applied to the score marking. These drawbacks are circumvented with the tablets of the present invention.

In GB-A-2,047,095 a pentagonal tablet is disclosed with scores which are situated in such a manner that a corner has to be broken from the tablet to get a third of the tablet.

The present invention provides a tablet structure that is readily divisible into sub-dosage units of two or three equal parts, said tablet comprising (i) an equilateral or isosceles triangle shape, (ii) two opposite triangular planar surfaces each having scoremarkings thereon, (iii) the scoremarkings on one surface consisting of a first scoremarking extended perpendicularly from the midpoint of one edge of the triangle and a second scoremarking positioned perpendicular to the first scoremarking at its distal end, wherein the length of the first scoremarking is selected such that the two perpendicular scoremarkings divide the tablet into three equal parts and (iv) a single scoremarking on the second surface dividing the tablet into two equal parts, wherein the scoremarking on the second surface is located to extend from an angle adjacent to the edge from which the first scoremarking on the opposite side extended or located to extend from the midpoint of the same edge, from which the first scoremarking, on the opposite side extended to the midpoint of the opposite angle.

It is easy to see that in choosing a triangular tablet shape scoremarkings can be positioned in a manner that the width of the tablet after breaking off one third remains the same, so that the ease of handling is not diminished.

The positioning is particularly easy when the three angles of the triangular shape are equal i.e. 60 degrees.

This embodiment gives the additional advantage of providing a tablet which can be easily swallowed, especially when the corners are rounded.

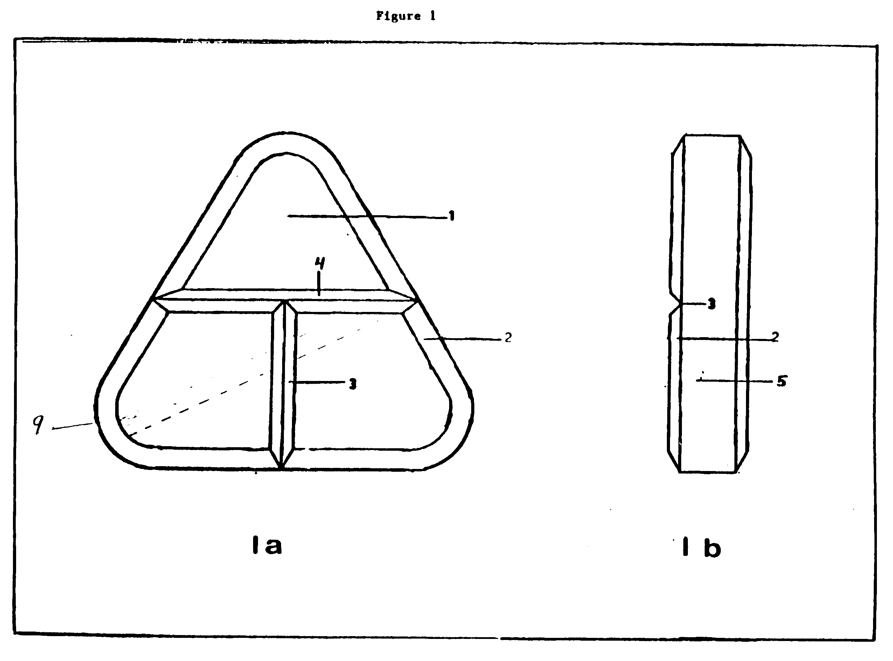

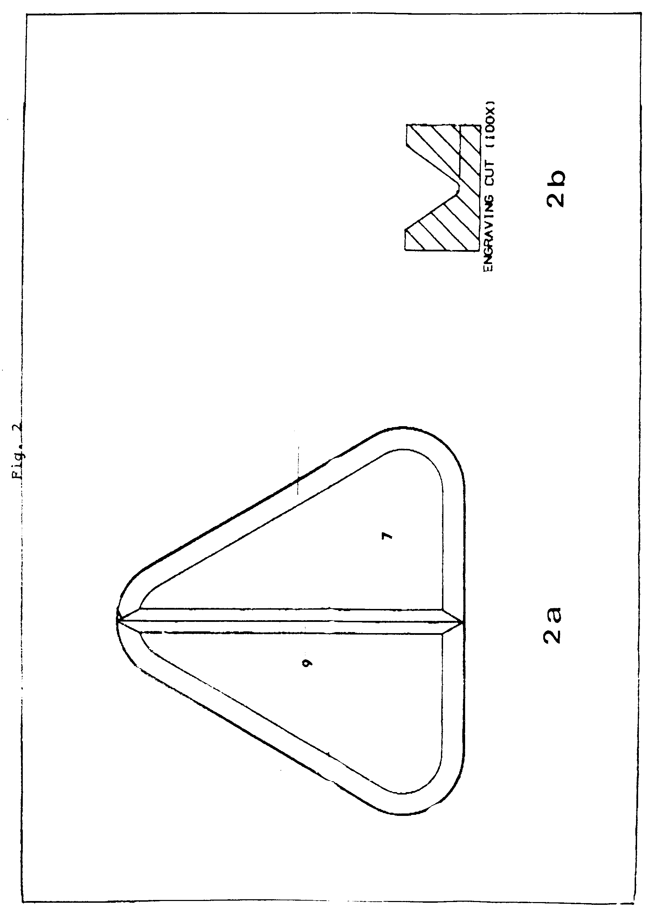

A tablet structure with three equal angles with especially positioned scoremarkings for breaking the tablet into equal bisectional and trisectional sub-dosage units is represented in figs. 1 and 2.

One tablet surface (fig. 1) comprises a first scoremarking extending from the midpoint of one of the sides of the triangle, up to a distance equivalent to one-half of the length of that side and a second scoremarking which is positioned at the end of the first scoremarking perpendicularly to it.

The other planar surface comprises a scoremarking extending from the angle opposite the side where the first scoremarking extends from midpoint, up to said midpoint.

If the scoremarkings are positioned in this manner, the first scoremarking is parallel to the one scoremarking on the other planar surface. This enhances the ease in breaking the tablet into halves. However, for breaking the tablet into thirds, it always has to be broken at the second scoremarking before breaking it at the other (parallel) scoremarkings. Mistakes are liable to be made, because of the parallel scoremarking on the other side the tablet which will be easily accidentally broken into halves. Therefore another useful embodiment comprises the same two scoremarkings as described hereinbefore on one planar surface, and on the other planar surface a scoremarking which extends from the angle adjacent to the side where the first scoremarking extends from midpoint, up to the midpoint on the side opposite said angle.

Such a tablet will still be readily dividable into halves and thirds, but errors in choosing the wrong scoremarking are avoided.

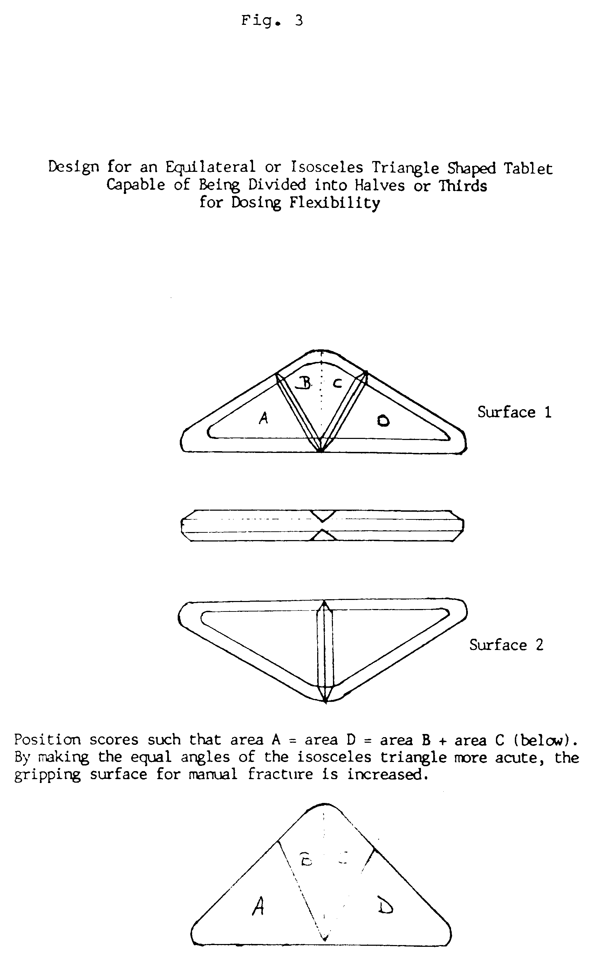

Another embodiment of the triangular tablet according to the invention is shown in fig. 3.

This figure shows a design for an equilateral or isosceles shaped tablet which can be devided into halves or thirds for flexible dosing. The scores on the tablet are positioned in such a way that the tablet on one side is devided into two equal parts and on the other side into three equal parts. The gripping surface for manual fracture of the tablet with the shape of an isosceles triangle may be increased by making the two equal angles more acute.

The two planar surfaces may be either flat, concave or convex. Usually these surfaces will be joined at the edges by more or less vertical walls, although this is not absolutely necessary if both surfaces are concave or if one surface is convex and the other is concave, wherein the edges of the tablet are nearly semi-circular.

If an embodiment including approximately vertical walls is chosen, then the planar surfaces may include bevel edges. For ease of production the scoremarkings may have an indentation depth to the line defining the bevel edges. The bevel edge angle may be any angle between 10 and 80; typically 30 to 60 degrees.

For ease of swallowing the angles of the triangular tabletstructure may be rounded off.

The scoremarkings may vary widely in size and shape. However, good results have been obtained with scoremarkings having a V-groove angle of 40 to 90 degrees, with a groove depth of 1/20 to 1/2 of the thickness of the tablet.

Of course, if both surfaces comprise parallel scoremarkings as represented in figs. 1 and 2, the groove depth should be chosen smaller than 1/2 of the width of the tablet.

Tablets of the invention may comprise a variety of ingredients, which are well known to the man skilled in the art.

They include among others, fillers, lubricants, carriers, flavouring ingredients and of course active pharmaceutical ingredients.

Many pharmaceutical active ingredients are suitable to be incorporated into multi-dosage tablets, such as inflammatory agents, antidepressive agents and anaesthetics.

The invention will be more specifically described in one of its embodiments in figs. 1 a,b and 2 a,b.
Fig. 1a is a top view of a tablet according to the invention.
Fig. 1b is a side view of the same tablet.
Fig. 2 is a bottom view of the tablet of fig. 1.
Fig. 3 is another embodiment of a tablet according to the invention.

In fig. 1a, (1) indicates the planar surface, with bevel edges (2) at the borders and with scoremarking (3) extending perpendicularly from the midpoint of the lower side up to a distance approximately one-half the length of said side where it meets score marking (5) which crosses the tablet transversely.

Fig. 1b shows a side view wherein (6) is the approximately vertical side wall with bevel edge (2) at its border and scoremarking (3) in the middle.

Fig. 2. shows the planar surface (7) of the bottom with bevel edges (8) and scoremarking (9).

Fig. 3 shows another triangular tablet with the scoremarkings positioned as indicated. The position of the scoremarkings is such that area A equals area D and equals area B + C.

The tablets may be made by known techniques for forming tablets. Such techniques are disclosed in Chase et al, Remington's Pharmaceutical Sciences. pp. 1553-1576 (16th ed. 1980). The invention thus includes methods of making the tablets involving adapting known techniques to the demands of the inventive tablets. Generally these adaptations will be modifications to the two steel punches within a steel die cavity used to form the tablets.

## Claims (Claims for the following Contracting State(s): AT, BE, CH, LI, DE, DK, FR, GB, IT, LU, NL, SE)

1. A tablet structure that is readily divisible into sub-dosage units of two or three equal parts, said tablet comprising (i) an equilateral or isosceles triangle shape, (ii) two opposite triangular planar surfaces each having scoremarkings thereon, (iii) the scoremarkings on one surface consisting of a first scoremarking extended perpendicularly from the midpoint of one edge of the triangle and a second scoremarking positioned perpendicular to the first scoremarking at its distal end, wherein the length of the first scoremarking is selected such that the two perpendicular scoremarkings divide the tablet into three equal parts and (iv) a single scoremarking on the second surface dividing the tablet into two equal parts, wherein the scoremarking on the second surface is located to extend from an angle adjacent to the edge from which the first scoremarking on the opposite side extended or located to extend from the midpoint of the same edge, from which the first scoremarking, on the opposite side extended to the midpoint of the opposite angle.

2. A tablet structure according to claim 1, wherein the scoremarkings have a depth of from 1/20 to ½ of the thickness of the tablet.

3. A tablet structure according to claim 1, having bevel edges and wherein the scoremarkings have an indentation depth to a line defining the bevel edges.

4. A tablet structure according to claim 3, wherein the bevel edge angle is in the range from 10 to 80 degrees from the adjacent surface.

5. A tablet structure according to claim 4, wherein the bevel edge angle is in the range from 30 to 60 degrees.

6. A tablet structure according to claim 1, wherein the scoremarkings have a V-groove angle of from 40 degrees to 90 degrees, and a groove depth of from 1/20 to ½ of the thickness of the tablet.

## Claims (Claims for the following Contracting State(s): ES, GR)

1. A tablet structure that is readily divisible into sub-dosage units of two or three equal parts, said tablet comprising (i) an equilateral or isosceles triangle shape, (ii) two opposite triangular planar surfaces each having scoremarkings thereon, (iii) the scoremarkings on one surface consisting of a first scoremarking extended perpendicularly from the midpoint of one edge of the triangle and a second scoremarking positioned perpendicular to the first scoremarking at its distal end, wherein the length of the first scoremarking is selected such that the two perpendicular scoremarkings divide the tablet into three equal parts and (iv) a single scoremarking on the second surface dividing the tablet into two equal parts, wherein the scoremarking on the second surface is located to extend from an angle adjacent to the edge from which the first scoremarking on the opposite side extended or located to extend from the midpoint of the same edge, from which the first scoremarking, on the opposite side extended to the midpoint of the opposite angle.

2. A tablet structure according to claim 1, wherein the scoremarkings have a depth of from 1/20 to ½ of the thickness of the tablet.

3. A tablet structure according to claim 1, having bevel edges and wherein the scoremarkings have an indentation depth to a line defining the bevel edges.

4. A tablet structure according to claim 3, wherein the bevel edge angle is in the range from 10 to 80 degrees from the adjacent surface.

5. A tablet structure according to claim 4, wherein the bevel edge angle is in the range from 30 to 60 degrees.

6. A tablet structure according to claim 1, wherein the scoremarkings have a V-groove angle of from 40 degrees to 90 degrees, and a groove depth of from 1/20 to ½ of the thickness of the tablet.

7. A method for the manufacture of a tablet structure that is readily divisible into sub-dosage units of two or three equal parts, said tablet comprising (i) an equilateral or isosceles triangle shape, (ii) two opposite triangular planar surfaces each having scoremarkings thereon, (iii) the scoremarkings on one surface consisting of a first scoremarking extended perpendicularly from the midpoint of one edge of the triangle and a second scoremarking positioned perpendicular to the first scoremarking at its distal end, wherein the length of the first scoremarking is selected such that the two perpendicular scoremarkings divide the tablet into three equal parts and (iv) a single scoremarking on the second surface dividing the tablet into two equal parts, wherein the scoremarking on the second surface is located to extend from an angle adjacent to the edge from which the first scoremarking on the opposite side extended or located to extend from the midpoint of the same edge, from which the first scoremarking, on the opposite side extended to the midpoint of the opposite angle, characterized in that the method comprises known techniques of making the tablet structures and use of two steel punches within a steel die cavity, modified in a manner to form the tablet structures.

8. The method according to claim 7, wherein the scoremarkings have a depth of from 1/20 to ½ of the thickness of the tablet, and preferably a V-groove angle of from 40 degrees to 90 degrees.

9. The method according to claim 7, wherein the tablet structures have bevel edges of which the bevel edge angle is preferably in the range from 10 to 80 degrees from the adjacent surface, and wherein the scoremarkings have an indentation depth to a line defining the bevel edges.

10. The method according to claim 7, wherein the tablet structures have bevel edges of which the bevel edge angle is in the range from 30 to 60 degrees from the adjacent surface, and wherein the scoremarkings have an indentation depth to a line defining the bevel edges.

## Patentansprüche (Patentansprüche für folgende(n) Vertragsstaat(en): AT, BE, CH, LI, DE, DK, FR, GB, IT, LU, NL, SE)

1. Tablettenstruktur, die leicht in Teildosiseinheiten von zwei oder drei gleichen Teilen geteilt werden kann, wobei diese Tablette (i) eine gleichseitige oder gleichschenklige Dreieckform umfasst, (ii) zwei einander gegenüberliegende dreieckige ebene Oberflächen, von denen jede mit Kerbenmarkierungen versehen ist, (iii) wobei die Kerbenmarkierungen auf einer Oberfläche aus einer ersten Kerbenmarkierung besteht, die sich senkrecht vom Mittelpunkt einer Kante des Dreiecks aus erstreckt, und eine zweite Kerbenmarkierung, die an ihrem distalen Ende senkrecht zur ersten Kerbenmarkierung positioniert ist, wobei die Länge der ersten Kerbenmarkierung derart gewählt wird, dass die zwei senkrechten Kerbenmarkierungen die Tablette in drei gleiche Teile teilen, und (iv) eine einzige Kerbenmarkierung auf der zweiten Oberfläche, welche die Tablette in zwei gleiche Teile teilt, wobei die Kerbenmarkierung auf der zweiten Oberfläche so angeordnet ist, das sie sich von einer Ecke aus erstreckt, die an die Kante grenzt, von der aus sich die erste Kerbenmarkierung auf der gegenüberliegenden Seite erstreckt, oder so angeordnet ist, dass sie sich vom Mittelpunkt der gleichen Kante aus erstreckt, von der sich die erste Kerbenmarkierung auf der gegenüberliegenden Seite zum Mittelpunkt der gegenüberliegenden Ecke erstreckt.

2. Tablettenstruktur nach Anspruch 1, wobei die Kerbenmarkierungen eine Tiefe von 1/20 bis ½ der Tablettendicke aufweisen.

3. Tablettenstruktur nach Anspruch 1, umfassend schräge Kanten, wobei die Kerbenmarkierungen eine Vertiefung bis zu einer Linie aufweisen, welche die schrägen Kanten definieren.

4. Tablettenstruktur nach Anspruch 3, wobei der Winkel der schrägen Kante im Bereich von 10 bis 80 Grad von der angrenzenden Oberfläche liegt.

5. Tablettenstruktur nach Anspruch 4, wobei der Winkel der schrägen Kante im Bereich von 30 bis 60 Grad liegt.

6. Tablettenstruktur nach Anspruch 1, wobei die Kerbenmarkierungen einen V-Nutenwinkel von 40 bis 90 Grad aufweisen und eine Nutentiefe von 1/20 bis ½ der Tablettendicke.

## Patentansprüche (Patentansprüche für folgende(n) Vertragsstaat(en): ES, GR)

1. Tablettenstruktur, die leicht in Teildosiseinheiten von zwei oder drei gleichen Teilen geteilt werden kann, wobei diese Tablette (i) eine gleichseitige oder gleichschenklige Dreieckform umfasst, (ii) zwei einander gegenüberliegende dreieckige ebene Oberflächen, von denen jede mit Kerbenmarkierungen versehen ist, (iii) wobei die Kerbenmarkierung auf einer Oberfläche aus einer ersten Kerbenmarkierung besteht, die sich senkrecht vom Mittelpunkt einer Kante des Dreiecks aus erstreckt, und eine zweite Kerbenmarkierung, die an ihrem distalen Ende senkrecht zur ersten Kerbenmarkierung positioniert ist, wobei die Länge der ersten Kerbenmarkierung derart gewählt wird, dass die zwei senkrechten Kerbenmarkierungen die Tablette in drei gleiche Teile teilen, und (iv) eine einzige Kerbenmarkierung auf der zweiten Oberfläche, welche die Tablette in zwei gleiche Teile teilt, wobei die Kerbenmarkierung auf der zweiten Oberfläche so angeordnet ist, das sie sich von einer Ecke aus erstreckt, die an die Kante grenzt, von der aus sich die erste Kerbenmarkierung auf der gegenüberliegenden Seite erstreckt, oder so angeordnet ist, dass sie sich vom Mittelpunkt der gleichen Kante aus erstreckt, von der sich die erste Kerbenmarkierung auf der gegenüberliegenden Seite zum Mittelpunkt der gegenüberliegenden Ecke erstreckt.

2. Tablettenstruktur nach Anspruch 1, wobei die Kerbenmarkierungen eine Tiefe von 1/20 bis ½ der Tablettendicke aufweisen.

3. Tablettenstruktur nach Anspruch 1, umfassend schräge Kanten, wobei die Kerbenmarkierungen eine Vertiefung bis zu einer Linie aufweisen, welche die schrägen Kanten definiert.

4. Tablettenstruktur nach Anspruch 3, wobei der Winkel der schrägen Kante im Bereich von 10 bis 80 Grad von der angrenzenden Oberfläche liegt.

5. Tablettenstruktur nach Anspruch 4, wobei der Winkel der schrägen Kante im Bereich von 30 bis 60 Grad liegt.

6. Tablettenstruktur nach Anspruch 1, wobei die Kerbenmarkierungen einen V-Nutenwinkel von 40 bis 90 Grad aufweisen, und eine Nutentiefe von 1/20 bis ½ der Tablettendicke.

7. Verfahren zum Herstellen einer Tablettenstruktur, die leicht in Teildosiseinheiten von zwei oder drei gleichen Teilen geteilt werden kann, wobei diese Tablette (1) eine gleichseitige oder gleichschenklige Dreieckform umfasst, (ii) zwei einander gegenüberliegende dreieckige ebene Oberflächen, von denen jede mit Kerbenmarkierungen versehen ist, (iii) wobei die Kerbenmarkierungen auf einer Oberfläche aus einer ersten Kerbenmarkierung besteht, die sich senkrecht vom Mittelpunkt einer Kante des Dreiecks aus erstreckt, und eine zweite Kerbenmarkierung, die an ihrem distalen Ende senkrecht zur ersten Kerbenmarkierung posttioniert ist, wobei die Länge der ersten Kerbenmarkierung derart gewählt wird, dass die zwei senkrechten Kerbenmarkierungen die Tablette in drei gleiche Teile teilen, und (iv) eine einzige Kerbenmarkierung auf der zweiten Oberfläche, welche die Tablette in zwei gleiche Teile teilt, wobei die Kerbenmarkierung auf der zweiten Oberfläche so angeordnet ist, das sie sich von einer Ecke aus erstreckt, die an die Kante grenzt, von der aus sich die erste Kerbenmarkierung auf der gegenüberliegenden Seite erstreckt, oder so angeordnet ist, dass sie sich vom Mittelpunkt der gleichen Kante aus erstreckt, von der sich die erste Kerbenmarkierung auf der gegenüberliegenden Seite zum Mittelpunkt der gegenüberliegenden Ecke erstreckt, dadurch gekennzeichnet, dass das Verfahren bekannte Techniken zum Herstellen der Tablettenstrukturen umfasst und Gebrauch macht von zwei Stahlstempeln in einer Stahlgesenkvertiefung, die derart abgeändert sind, um die Tablettenstruktur bilden.

8. Verfahren nach Anspruch 7, wobei die Kerbenmarkierungen eine Tiefe von 1/20 bis ½ der Tablettendicke aufweisen, und vorzugsweise einen V-Nutenwinkel von 40 bis 90 Grad.

9. Verfahren nach Anspruch 7, wobei die Tablettenstrukturen schräge Kanten aufweisen, wobei der Winkel der schrägen Kante vorzugsweise im Bereich von 10 bis 80 Grad von der angrenzenden Oberfläche liegt, und wobei die Kerbenmarkierungen eine Vertiefung bis zu einer Linie aufweisen, welche die schrägen Kanten definiert.

10. Verfahren nach Anspruch 7, wobei die Tablettenstrukturen schräge Kanten aufweisen, wobei der Winkel der schrägen Kanten im Bereich von 30 bis 60 Grad von der angrenzenden Oberfläche liegt, und wobei die Kerbenmarkierungen eine Vertiefung bis zu einer Linie aufweisen, welche die schrägen Kanten definiert.

## Revendications (Revendications pour l'(les) Etat(s) contractant(s) suivant(s): AT, BE, CH, LI, DE, DK, FR, GB, IT, LU, NL, SE)

1. Une structure de tablette ou de comprimé qui est aisément divisible en unités de sous-dosage constituées de deux ou trois parties égales, ladite tablette comprenant:
(i) une forme de triangle équilatéral ou isocèle;
(ii) deux surfaces triangulaires opposées de forme générale plane munies chacune de marquage d'affaiblissement;
(iii) les marquages d'affaiblissement sur l'une des surfaces consistant en un premier marquage d'affaiblissement s'étendant perpendiculairement à partir du point médian d'un des côtés du triangle, et en un second marquage d'affaiblissement disposé perpendiculairement au premier marquage d'affaiblissement à son extrémité distale, dans lequel la longueur du premier marquage d'affaiblissement est choisie de telle façon que les deux marquages d'affaiblissement perpendiculaires divisent la tablette en trois parties égales; et
(iv) un marquage d'affaiblissement unique sur la seconde surface divisant la tablette en deux parties égales, dans laquelle le marquage d'affaiblissement sur la seconde surface est disposé de façon à s'étendre à partir d'un angle adjacent au côté à partir duquel le premier marquage d'affaiblissement sur le côté opposé s'étend ou est disposé de façon à s'étendre à partir du point médian du même côté, à partir duquel le premier marquage d'affaiblissement, du côté opposé, est étendu jusqu'au point médian de l'angle opposé.

2. Une structure de tablette selon la revendication 1, dans laquelle les marquages d'affaiblissement présentent une profondeur comprise entre 1/20ème et 1/2 de l'épaisseur de la tablette.

3. Une structure de tablette selon la revendication 1, présentant des côtés chanfreinés et dans laquelle les marquages d'affaiblissement présentent une profondeur d'indentation s'étendant jusqu'à une ligne définissant les côtés chanfreinés.

4. Une structure de tablette selon la revendication 3, dans laquelle l'angle des côtés chanfreinés est compris entre 10 et 80 degrés à partir de la surface adjacente.

5. Une structure de tablette selon la revendication 4, dans laquelle l'angle de côté chanfreiné est compris entre 30 et 60 degrés.

6. Une structure de tablette selon la revendication 1, dans laquelle les marquages d'affaiblissement présentent un angle de rainure en V compris entre 40 et 90 degrés, et une profondeur de rainure comprise entre 1/20ème et 1/2 de l'épaisseur de la tablette.

## Revendications (Revendications pour l'(les) Etat(s) contractant(s) suivant(s): ES, GR)

1. Une structure de tablette ou de comprimé qui est aisément divisible en unités de sous-dosage constituées de deux ou trois parties égales, ladite tablette comprenant:
(i) une forme de triangle équilatéral ou isocèle;
(ii) deux surfaces triangulaires opposées de forme générale plane munies chacune de marquage d'affaiblissement;
(iii) les marquages d'affaiblissement sur l'une des surfaces consistant en un premier marquage d'affaiblissement s'étendant perpendiculairement à partir du point médian d'un des côtés du triangle, et en un second marquage d'affaiblissement disposé perpendiculairement au premier marquage d'affaiblissement à son extrémité distale, dans lequel la longueur du premier marquage d'affaiblissement est choisie de telle façon que les deux marquages d'affaiblissement perpendiculaires divisent la tablette en trois parties égales; et
(iv) un marquage d'affaiblissement unique sur la seconde surface divisant la tablette en deux parties égales, dans laquelle le marquage d'affaiblissement sur la seconde surface est disposé de façon à s'étendre à partir d'un angle adjacent au côté à partir duquel le premier marquage d'affaiblissement sur le côté opposé s'étend ou est disposé de façon à s'étendre à partir du point médian du même côté, à partir duquel le premier marquage d'affaiblissement, du côté opposé, est étendu jusqu'au point médian de l'angle opposé.

2. Une structure de tablette selon la revendication 1, dans laquelle les marquages d'affaiblissement présentent une profondeur comprise entre 1/20ème et 1/2 de l'épaisseur de la tablette.

3. Une structure de tablette selon la revendication 1, présentant des côtés chanfreinés et dans laquelle les marquages d'affaiblissement présentent une profondeur d'indentation s'étendant jusqu'à une ligne définissant les côtés chanfreinés.

4. Une structure de tablette selon la revendication 3, dans laquelle l'angle des côtés chanfreinés est compris entre 10 et 80 degrés à partir de la surface adjacente.

5. Une structure de tablette selon la revendication 4, dans laquelle l'angle de côté chanfreiné est compris entre 30 et 60 degrés.

6. Une structure de tablette selon la revendication 1, dans laquelle les marquages d'affaiblissement présentent un angle de rainure en V compris entre 40 et 90 degrés, et une profondeur de rainure comprise entre 1/20ème et 1/2 de l'épaisseur de la tablette.

7. Un procédé pour la fabrication d'une structure de tablette qui est aisément divisible en unités de sous-dosage constituées de deux ou trois parties égales, ladite tablette comprenant:
(i) une forme de triangle équilatéral ou isocèle;
(ii) deux surfaces triangulaires opposées de forme générale plane munies chacune de marquage d'affaiblissement;
(iii) les marquages d'affaiblissement sur l'une des surfaces consistant en un premier marquage d'affaiblissement s'étendant perpendiculairement à partir du point médian d'un des côtés du triangle, et en un second marquage d'affaiblissement disposé perpendiculairement au premier marquage d'affaiblissement à son extrémité distale, dans lequel la longueur du premier marquage d'affaiblissement est choisie de telle façon que les deux marquages d'affaiblissement perpendiculaires divisent la tablette en trois parties égales; et
(iv) un marquage d'affaiblissement unique sur la seconde surface divisant la tablette en deux parties égales, dans laquelle le marquage d'affaiblissement sur la seconde surface est disposé de façon à s'étendre à partir d'un angle adjacent au côté à partir duquel le premier marquage d'affaiblissement sur le côté opposé s'étend ou est disposé de façon à s'étendre à partir du point médian du même côté, à partir duquel le premier marquage d'affaiblissement, du côté opposé, est étendu jusqu'au point médian de l'angle opposé,
caractérisé en ce que le procédé comporte des techniques connues de fabrication de structure de tablette et d'utilisation de deux poinçons en acier à l'intérieur d'une cavité de poinçonnage en acier, modifiée de façon à réaliser les structures de tablettes.

8. Le procédé selon la revendication 7, dans lequel les marquages d'affaiblissement présentent une profondeur comprise entre 1/20ème et 1/2 de l'épaisseur de la tablette, et préférablement un angle de rainure en V compris entre 40 degrés et 90 degrés.

9. Le procédé selon la revendication 7, dans lequel les structures de tablettes comportent des côtés chanfreinés dont l'angle de face chanfreinée est de préférence compris entre 10 et 80 degrés à partir de la surface adjacente, et dans lequel les marquages d'affaiblissement présentent une profondeur d'indentation s'étendant jusqu'à une ligne définissant les côtés chanfreinés.

10. Le procédé selon la revendication 7, dans lequel les structures de tablettes comportent des côtés chanfreinés dont l'angle de côté chanfreiné est compris entre 30 et 60 degrés à partir de la surface adjacente, et dans lequel les marquages d'affaiblissement présentent une profondeur d'indentation s'étendant jusqu'à une ligne définissant les côtés chanfreinés.
